# EUROPEAN PATENT APPLICATION

(11) **EP 2 412 326 A2**
(43) Date of publication of application: **01.02.2012**
(21) Application number: 11175454.5
(22) Date of filing: 26.07.2011
(51) Int. Cl.: A61B 17/34, A61M 29/00

(54) **Laterally expanding surgical dilator**

(30) Priority: 26.07.2010 US 843079
(71) Applicant: Warsaw Orthopedic, Inc., Warsaw, IN 46581 (US)
(72) Inventor: Mire, David A., Cordova, TN Tennessee 38018 (US); Sebastian, Kelli N., Arlington, TN Tennessee 38002 (US); Wheeler, Paul F., Hernando, MS Mississippi 38632 (US)
(74) Representative: Hutchinson, Glenn Stanley

(57) **Abstract**

A surgical dilator capable of dilating an incision in a patient is disclosed. The surgical dilator includes a main body having a distal end and a proximal end. A locking cap is connected with the proximal end of the main body that is operable to lock the surgical dilator in a closed state for insertion into an incision and to unlock the surgical dilator to an open state after being inserted into the incision. A plurality of blades having a second proximal end is pivotally connected with the distal end of the main body and extends downwardly toward a second distal end. When the surgical dilator is in the closed state the blades taper downwardly and inwardly from the second proximal end toward the second distal end to form an insertion tip and when the surgical dilator is in the open state the blades are operable to laterally expand.

## Description

The present invention relates generally to percutaneous surgeries and more particularly, to devices for performing percutaneous, minimally invasive spinal surgeries.

Traditional surgical procedures for pathologies located deep within the body can cause significant trauma to the intervening tissues. These open procedures often require a long incision, extensive muscle stripping, prolonged retraction of tissues, denervation and devascularization of tissue. Most of these surgeries require a recovery room time of several hours and several weeks of post-operative recovery time due to the use of general anesthesia and the destruction of tissue during the surgical procedure. In some cases, these invasive procedures lead to permanent scarring and pain that can be more severe than the pain leading to the surgical intervention.

Minimally invasive alternatives such as arthroscopic techniques reduce pain, post-operative recovery time and the destruction of healthy tissue. Orthopedic surgical patients have particularly benefitted from minimally invasive surgical techniques. The site of pathology is accessed through portals rather than through a significant incision thus preserving the integrity of the intervening tissues. In some instances, these minimally invasive techniques require only local anesthesia. The avoidance of general anesthesia reduces post-operative recovery time and the risk of complications.

Minimally invasive surgical techniques are particularly desirable for spinal and neurosurgical applications because of the need for access to locations deep within the body and the danger of damage to vital intervening tissues. For example, a common open procedure for disc herniation, laminectomy followed by discectomy requires stripping or dissection of the major muscles of the back to expose the spine. In a posterior approach, tissue including spinal nerves and blood vessels around the dural sac, ligaments and muscle must be retracted to clear a channel from the skin to the disc. These procedures normally take at least one-two hours to perform under general anesthesia and require post-operative recovery periods of at least several weeks. In addition to the long recovery time, the destruction of tissue is a major disadvantage of open spinal procedures. This aspect of open procedures is even more invasive when the discectomy is accompanied by fusion of the adjacent vertebrae. Many patients are reluctant to seek surgery as a solution to pain caused by herniated discs and other spinal conditions because of the severe pain sometimes associated with the muscle dissection.

In order to reduce the post-operative recovery time and pain associated with spinal and other procedures, micro-surgical techniques have been developed. The objective of any minimally invasive procedure is to accomplish the same clinical objectives as the traditional, open surgery while minimizing soft tissue retraction. Existing sequential dilation processes consist of inserting multiple increasing diameter dilators until the correct diameter is achieved. A tubular retractor is then placed over the dilators and the dilators are then removed. The retractor is left in place with the surrounding muscle and tissue having been dilated out of the working space.

For some applications, it would be beneficial to be able to dilate an incision quickly without the use of multiple individual dilators. As such, a need exists for a device that will allow physicians to quickly dilate an incision without the use of multiple individual components.

### SUMMARY

According to one aspect of an embodiment of the present invention, a surgical dilator is disclosed that is capable of dilating an incision in a patient. The surgical dilator includes a main body having a distal end and a proximal end. A locking cap is connected with the proximal end of the main body that is operable to lock the surgical dilator in a closed state for insertion into an incision as well as to unlock the surgical dilator to an open state after being inserted into the incision. A plurality of blades having a second proximal end pivotally connected with the distal end of the main body and extend downwardly toward a second distal end. When the surgical dilator is in the closed state the blades taper downwardly and inwardly from the second proximal end toward the second distal end to form an insertion tip and when the surgical dilator is in the open state the blades are operable to laterally expand.

In a preferable embodiment, an expansion tube is sized to be inserted into an interior cavity defined by the main body and make contact with an interior surface of each of the blades. As the expansion tube is compressed into the interior cavity the expansion tube may cause the blades to laterally expand and dilate the incision. A width of each of the blades is preferably larger at the proximal end and tapers to a smaller width at the second distal end. In the closed state the blades may form a generally uniform cone-shaped blade. A collar may be positioned between the proximal end of the main body and an end of the locking cap. A plurality of locking pins may be connected with a lower surface of the collar and extends downwardly through a plurality of channels formed in the main body. In the closed state a distal end of each the locking pins may extend outwardly from the distal end of the main body and engage an aperture in a respective blade thereby forcing the blades into the closed state. In the open state a distal end of each the locking pins may be retracted away from the blades thereby allowing the blades to pivotally move in relation to the main body.

Another aspect of an embodiment of the present invention discloses a surgical dilator that is capable of dilating an incision in a patient. In this form, the surgical dilator includes a main body having a distal end and a proximal end. A locking cap is connected with the proximal end of the main body. A collar is positioned between the locking cap and the proximal end of the main body. A plurality of locking pins are connected with an end of the collar and positioned in a plurality of channels located in the main body. A distal end of the locking pins is operable to protrude outwardly from the channels on the distal end of the main body. A plurality of blades is movably connected with the distal end of the main body. The distal ends of the locking pins are operable to engage an aperture in the blades to force the blades into a closed state for inserting the blades into an incision.

In one form, the second distal ends of the locking pins are operable to be released from the blades so that the blades are operable to laterally expand. A plurality of springs may be included that have a first portion positioned in a set of apertures located on the proximal end of the main body and a second portion in engagement with the end of the collar. The springs may be operable to force the locking cap upwardly thereby causing the distal ends of the locking pins to disengage the aperture in the blades thereby releasing the blades from the closed state. A pin may be positioned in a proximal portion of the main body that extrudes through a control slot in a side surface of the locking cap. In one preferable embodiment, as the locking cap is rotated about the main body the control slot includes a vertical slot portion that becomes aligned with the pin thereby causing the springs to force the locking cap upwardly thereby disengaging the locking pins from the aperture.

In another form, the locking cap includes a conductive area and at least a lower portion of at least one of the blades includes a second conductive area. The second conductive area may be configured to transmit an electric signal to provide the surgical dilator with neural monitoring capabilities. The distal end of the main body may include a plurality of mounting arms and a proximal end of each of the blades includes an arm. The arms of the blades may be oriented between the mounting arms of the main body and the arms may be pivotally connected to the mounting arms with a plurality of pins. The pins may be inserted into the mounting arms and into the arms of the blades through a plurality of pin channels located on a side surface of the mounting arms. The surgical dilator may also include an expansion tube that is sized to be inserted into an interior cavity formed in the main body and into contact with an interior surface of the blades. As the expansion tube is compressed downwardly the blades may laterally expand when the blades are not in the closed state.

Yet a further aspect of an embodiment of the present invention discloses a surgical dilator that is capable of dilating an incision in a patient. The surgical dilator includes a main body having a distal end and a proximal end. A plurality of blades is connected with and extend downwardly from the distal end of the main body. The surgical dilator includes means for orienting the blades in a locked state in which the blades combine to form a generally uniform blade structure having a generally cone-shaped configuration. In addition, the surgical dilator further includes means for orienting the blades in an unlocked state in which the blades can be laterally expanded.

In one representative form, the means for orienting the blades in the locked state comprises a locking cap connected with the proximal end of the main body, a collar positioned between the proximal end and the locking cap having a plurality of locking pins connected therewith that extend through a plurality of channels in the main body to engage the blades to orient the blades in the locked state. In addition, the means for orienting the blades in the unlocked state may comprise a control slot on a side surface of the locking cap and a pin extending through the control slot that is located on the main body. When the pin is oriented in the control slot in a predetermined position the locking pins may disengage the blades thereby allowing the blades to laterally expand. A plurality of springs may be positioned between the proximal end of the main body and the collar to bias the locking pins in a position in which the blades are maintained in the unlocked state.

Related features, aspects, embodiments, objects and advantages of the present invention will be apparent from the following description.

### BRIEF DESCRIPTION OF THE DRAWINGS

FIG. 1 is a perspective view of a surgical dilator oriented in a closed or retracted state.
FIG. 2 is a perspective view of the surgical dilator illustrated in FIG. 1 oriented in an open or expanded state.
FIG. 3 is a perspective view of a portion of the surgical dilator illustrated in FIG. 1.
FIG. 4 is an end view of a main body of the surgical dilator illustrated in FIG. 1.
FIG. 5 is a perspective view of a representative blade of the surgical dilator illustrated in FIG. 1.
FIG. 6 is a perspective view of a portion of the surgical dilator illustrated in FIG. 1.
FIG. 7 is a perspective view of a portion of the surgical dilator illustrated in FIG. 1.
FIG. 8 is a perspective view of a portion of the surgical dilator illustrated in FIG. 1.
FIG. 9 is a top view of the surgical dilator illustrated in FIG. 1.
FIGS. 10a-10d is a perspective view of illustrative expansion tubes.
FIG. 11 is a perspective view of another representative surgical dilator.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any such alterations and further modifications in the illustrated devices, and such further applications of the principles of the invention as illustrated herein are contemplated as would normally occur to one skilled in the art to which the invention relates.

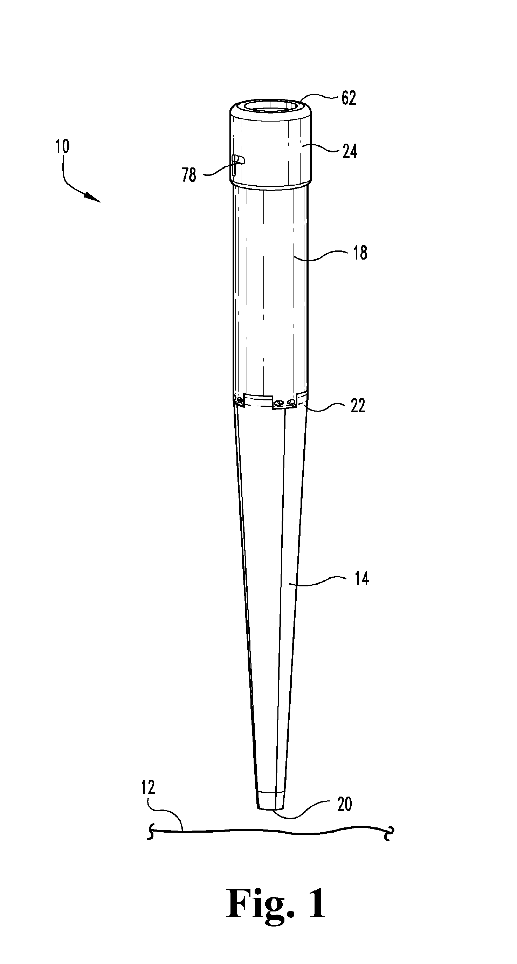

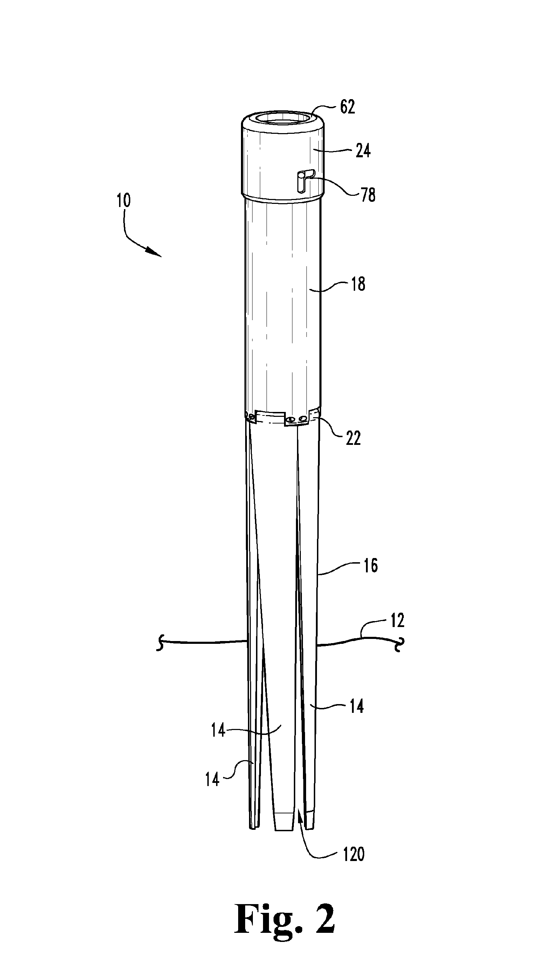

Referring collectively to Figs. 1 and 2, a soft tissue surgical dilator 10 is disclosed that is operable to dilate an incision 12 in a patient. As illustrated in Fig. 1, prior to insertion into the incision 12 to be dilated, the dilator 10 is oriented in a closed or retracted state. As set forth in greater detail below, the dilator 10 is biased in the closed state but can be unlocked to allow the dilator 10 to laterally expand. After being oriented in the incision 12 at a predetermined depth, the surgical dilator 10 is unlocked and then expansion tubes can be inserted into the interior of the dilator 10 to laterally expand a plurality of tapered blades 14 attached to a distal portion 16 of the dilator 10. In the closed state, the tapered blades 14 form a generally uniform cone-shaped dilator blade that includes a small insertion tip or vertex 20 that expands upwardly and outwardly to a base 22. In the illustrated form, while in the closed state the blades 14 form a generally uniform blade having a generally circular shaped cross-section along the horizontal plane of the blades 14. However, other forms are envisioned, for example the blades 14 could combine in the closed state to form an elliptical or oval shaped unitary insertion blade that laterally expands.

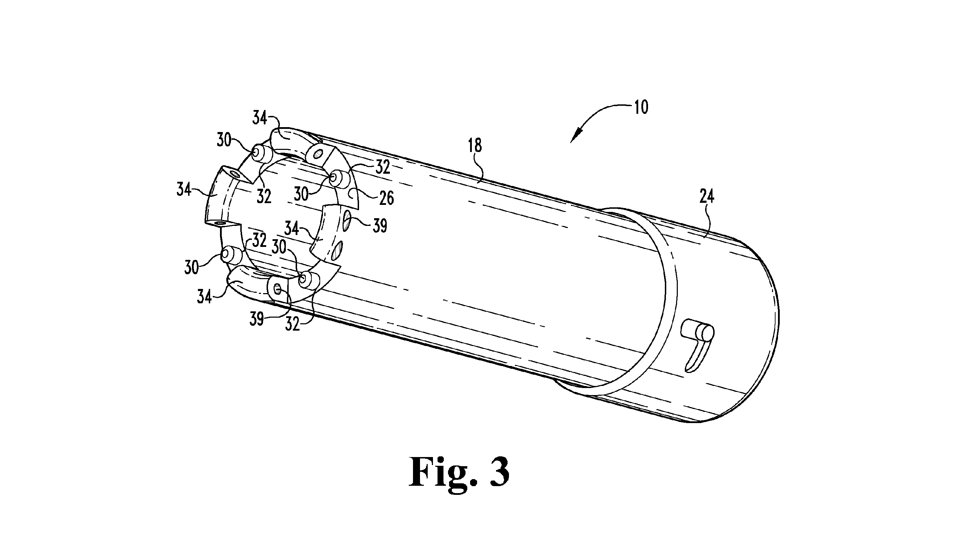

The dilator 10 includes a main body 18, a locking cap 24, and tapered blades 14. The locking cap 24 is used to lock the blades 14 in the closed state for insertion into the incision 12 and to release or unlock the blades 14 so that they can be expanded outwardly for the purpose of dilating the incision 12. Referring to Fig. 3, the blades 14 have been removed from a distal end 26 of the main body 18. A plurality of locking members or rods 30 extend outwardly from a plurality of channels 32 defined through the main body 18 while the blades 14 are oriented in the closed state. As set forth in greater detail below, the locking rods 30 are retracted in the channels 32 when the blades 14 are oriented in the released state for the purpose of dilating the incision 12.

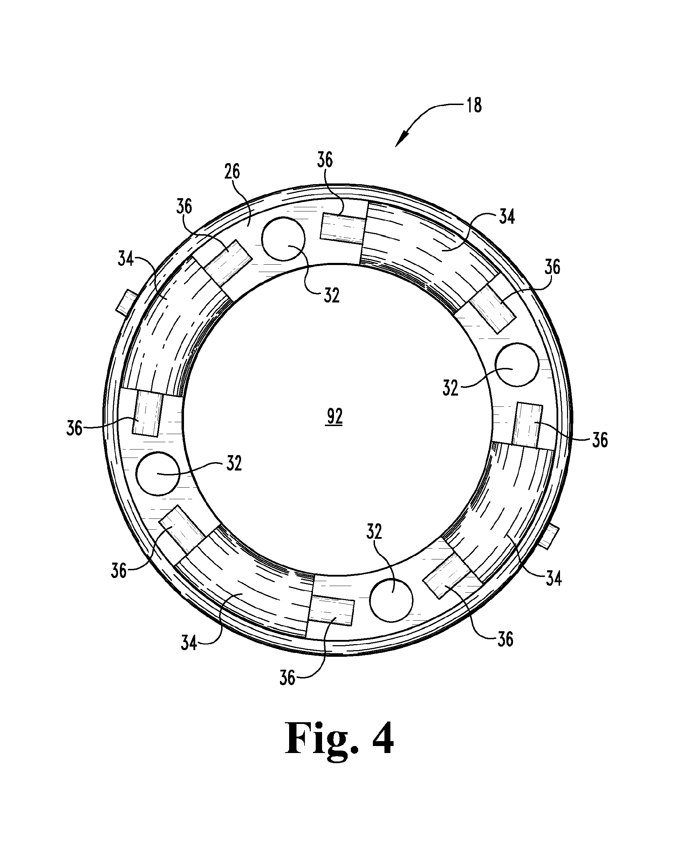

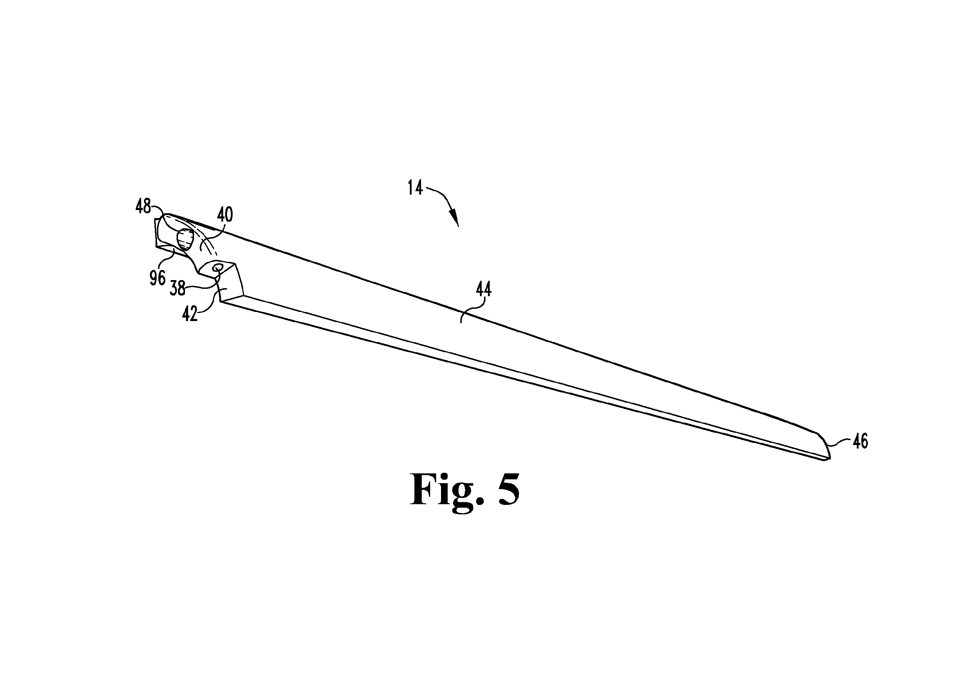

A plurality of blade mounting arms 34 extend outwardly from the distal end 26 of the main body 18. As illustrated in Figs. 3 and 4, the blade mounting arms 34 include a plurality of blade mounting pins 36 that are used to pivotally connect the blades 14 to the main body 18. The blade mounting pins 36 are inserted into the blade mounting arms 34 through a plurality of pin channels 39 that are bored or formed in the blade mounting arms 34. Referring to Fig. 5, each blade 14 includes a channel 38 in an arm 40 extending from a proximal end 42 of the blade 14. A portion of the mounting pins 36 that extend out of the blade mounting arms 34 are positioned in the channel 38 when the blades 14 are connected with the main body 18. As illustrated in Fig. 5, the blades 14 include a blade body 44 that extends from the proximal end 42 of the blade 14 to a distal end 46. The blade body 44 has a greater width at the proximal end 42 and tapers downwardly to a smaller width at the distal end 46. The blade body 44 and arms 40 have a generally curved cross-sectional shape along a horizontal plane. In the form having four blades 14, the generally curved cross-sectional shape comprises 90 degrees of a circle when viewed along the horizontal plane. Although four blades 14 are used in the illustrated form, it is envisioned that at least two or more blades 14 can be used in other forms.

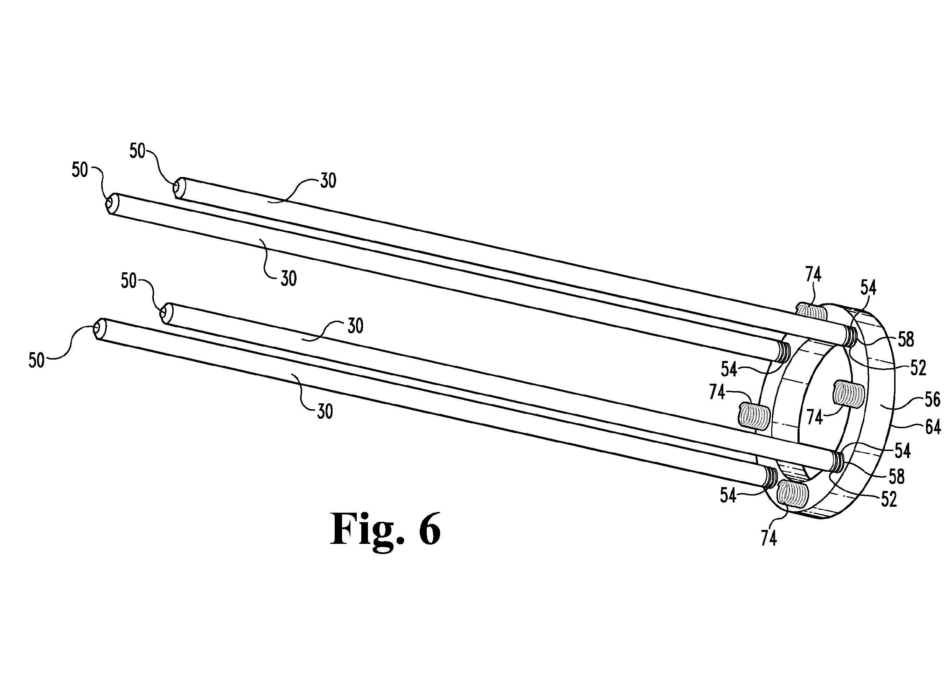

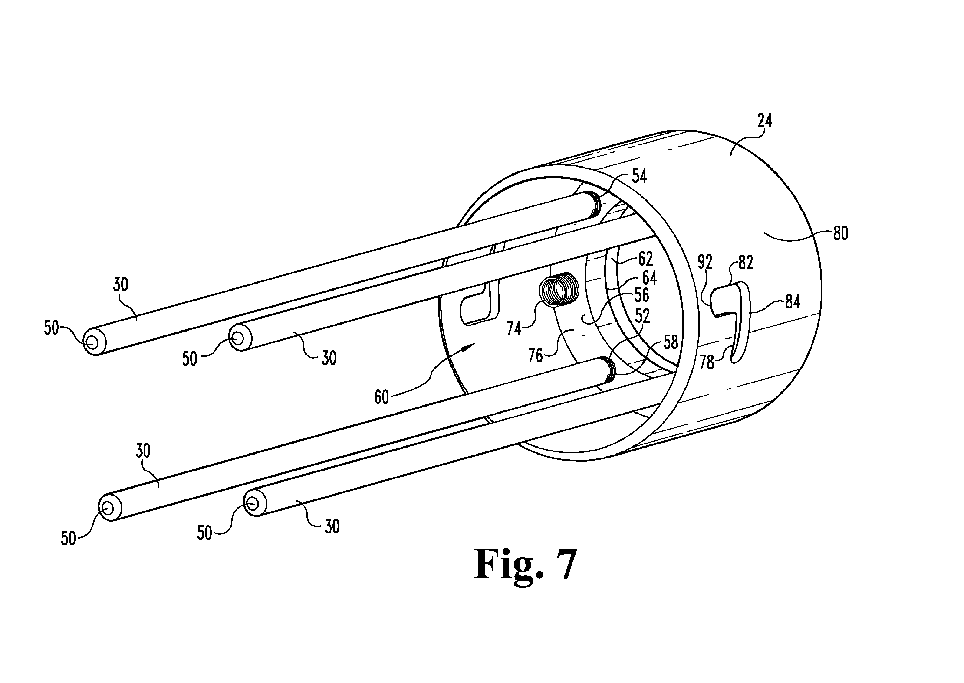

Referring collectively to Figs. 1, 3 and 5, while the blades 14 are oriented in the closed state, the locking rods 30 extend outwardly from the distal end 26 of the main body 18 and into a closing aperture 48 in each arm 40 of each respective blade 14. The locking rods 30 force the blades 14 to collapse on one another into the closed stated. Referring to Figs. 6 and 7, the locking rods 30 extend from a distal end 50 to a proximal end 52 and have a rod-like shape. In one form, the distal end 50 of the locking rods 30 is tapered to facilitate insertion into the closing aperture 48 in the arm 40 of each blade 14. The proximal end 52 of the locking rods 30 includes an external threaded portion 54. The surgical dilator 10 also includes a collar 56 that has a generally circular cross-sectional shape along a horizontal plane of the collar 56. The collar 56 includes a plurality of internally threaded apertures 58 that are sized to threadably engage the external threaded portion 54 of the locking rods 30. As such, the locking rods 30 are connected with the collar 56 via a threaded connection in this form, but other methods of connecting the locking rods 30 to the collar 56 could be used in other forms such as a press or friction fit, a weld, an adhesive or any other suitable connection means.

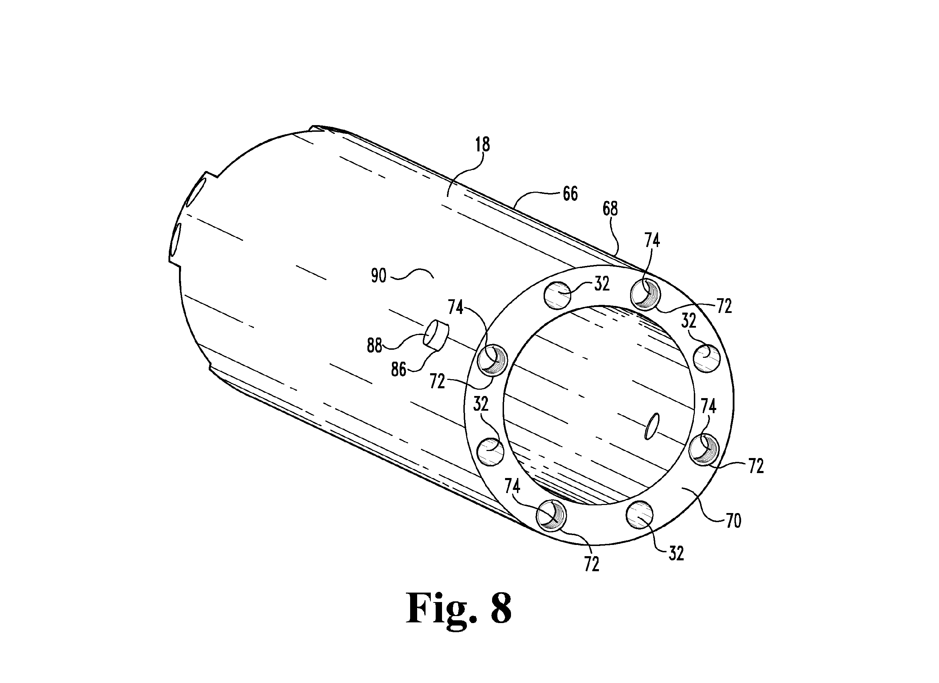

As illustrated in Fig. 7, the collar 56 is sized and configured to fit within an interior cavity 60 defined by the locking cap 24. The locking cap 24 has a generally tubular shape that includes an upper inwardly extending rim 62 to which an upper surface 64 of the collar 24 abuts or is connected with. Referring collectively to Figs. 1, 7 and 8, the interior cavity 60 of the locking cap 24 is sized to slide over an outer or exterior surface 66 of the main body 18. In particular, the locking cap 24 slides over a proximal end portion 68 of the main body 18. Prior to or simultaneously with the attachment of the locking cap 24 to the main body 18, the distal ends 50 of the locking pins 30 are aligned with the locking pin channels 32 and inserted into the locking pin channels 32 of the main body 18.

A distal end 70 of the main body 18 includes a plurality of spring apertures 72 that are sized and configured to receive a plurality of springs 74. When installed, the springs 74 exert an upward force on a lower surface 76 of the collar 56 thereby tending to bias the locking pins 30 in a retracted state. The locking cap 24 also includes at least one L-shaped control slot 78 on an outside surface 80 of the locking cap 24 that allows the locking cap 24 to be oriented in locked and unlocked positions. As previously set forth, in the unlocked position the blades 14 are free to expand outwardly to dilate the incision 12 and in the locked position the blades 14 are forced together in the closed state. Keeping the blades 14 locked in the closed state permits the blades 14 to be inserted without having to be concerned about the blades 14 wanting to separate as the blades 14 are inserted into the incision 12.

As set forth above, the springs 74 bias the locking cap 24 in an upward position by applying force to the lower surface 76 of the collar 56. The L-shaped control slot 78 comprises a vertical slot portion 82 and a horizontal slot portion 84. The distal end portion 68 of the main body 18 includes at least one cap pin aperture 86 that is sized to receive a locking cap pin 88 that is exposed on an outside surface 90 of the main body 18. Once the locking pins 30 are inserted into the main body 18 and the locking cap 24 is positioned over the distal end portion 68 of the main body 18, the locking cap pin 88 is inserted into the cap pin aperture 86 through the control slot 78 in the locking cap 24 thereby securing the locking cap 24 to the main body 18.

To unlock the blades 14, the locking cap 24 is rotated about the main body 18 so that the locking cap pin 88 is exposed to the vertical slot portion 82 of the control slot 78. The springs 74 then force the collar 56 and locking cap 24 upwardly thereby placing the locking pins 30 in a retracted position in which the locking pins 30 no longer force the blades 14 to be in the closed state. In particular, this causes the distal ends 50 of the locking pins 30 to be removed from the closing apertures 48 in the blades 14 thereby allowing them to pivotally move about the blade mounting pins 36. The locking cap pin 88 makes contact with a distal end 92 of the vertical slot portion 82 to stop the locking cap 24 and collar 56 from travelling upwardly on the distal end 68 of the main body 18 any further than necessary to unlock the locking pins 30. To lock the blades 14 back in a closed state, a user exerts a downward force on the locking cap 24 thereby causing the springs 74 to compress and then rotates the locking cap 24 horizontally to orient the locking cap pin 88 in the horizontal slot portion 84 of the control slot 78. Compression of the springs 74 causes the locking cap 24 to exert a downward force on the collar 56 thereby forcing the distal ends 50 of the locking pins 30 back into the closing apertures 48 of the blades 14. The orientation or alignment of the closing apertures 48 in the blades 14 forces the blades 14 to the closed state as the locking pins 30 are received in the closing apertures 48.

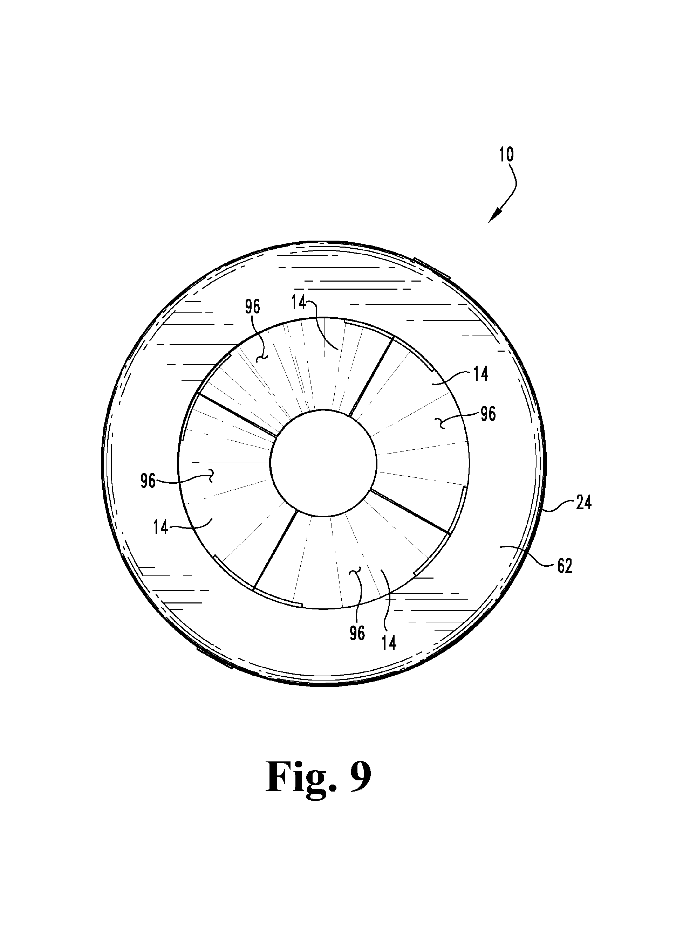

Referring to Figs. 1, 3 and 9, the main body 18 has a generally tubular shape defining a hollow interior 92. The locking cap 24 includes a rim 62 that also defines an opening 94 into the hollow interior 92 defined by the main body 18. While in the closed state, the blades 14 define a generally downwardly extending conically shaped uniform blade terminating at a small insertion tip 20. In the closed state, interiorly curved surfaces 96 of the blades 14 also define a hollow interior portion 98 of the surgical dilator 10. After being inserted into the incision 12 and placed in the unlocked position, the blades 14 of the surgical dilator 10 need to be expanded in order to further dilate the incision 12. As set forth in detail below, dilation of the incision 12 is obtained through the use of tapered tubes that are inserted into the hollow interior 92 of the main body 18 and the hollow interior portion 98 defined by the blades 14.

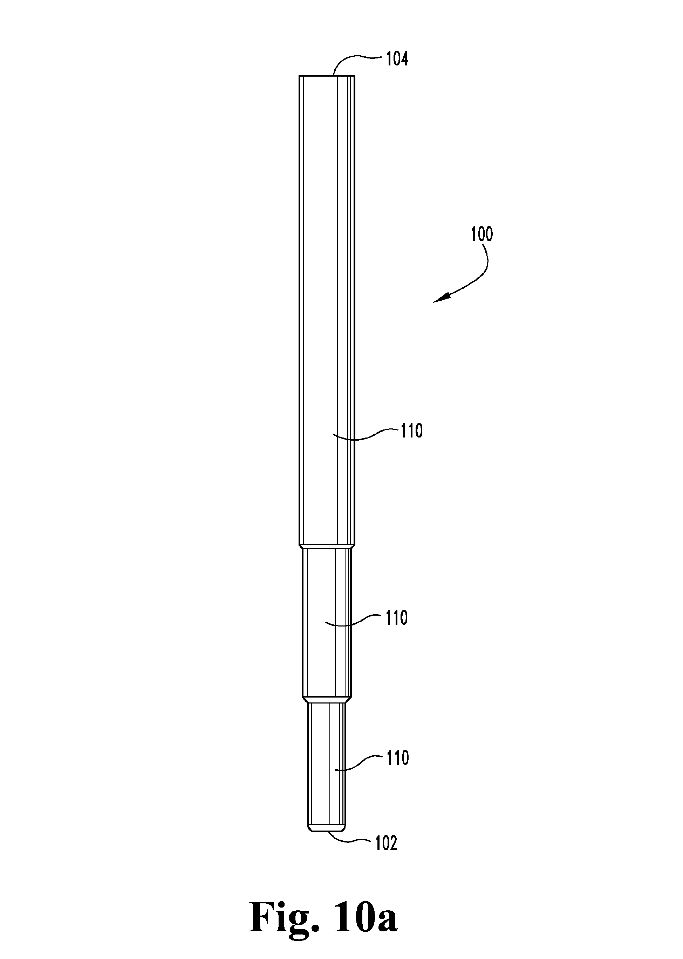

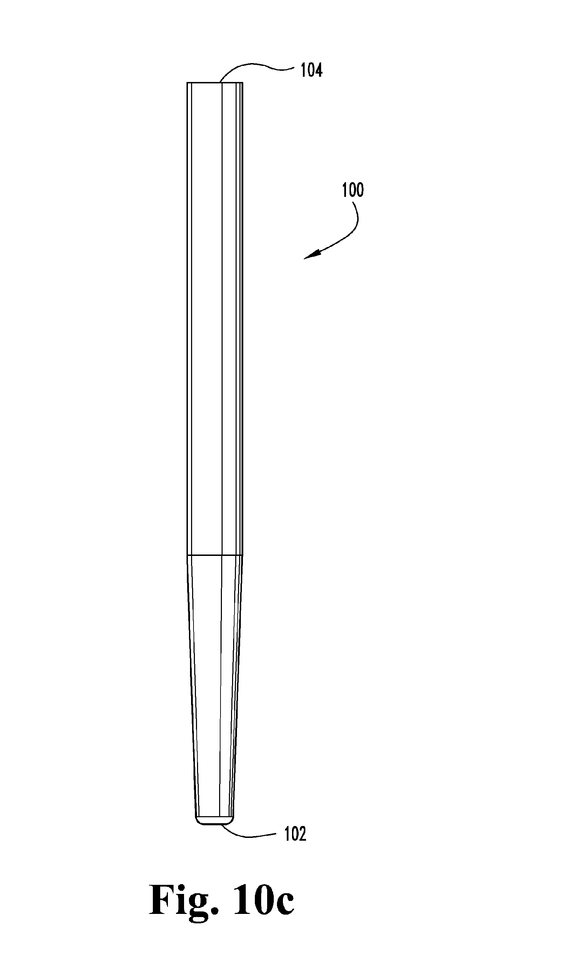

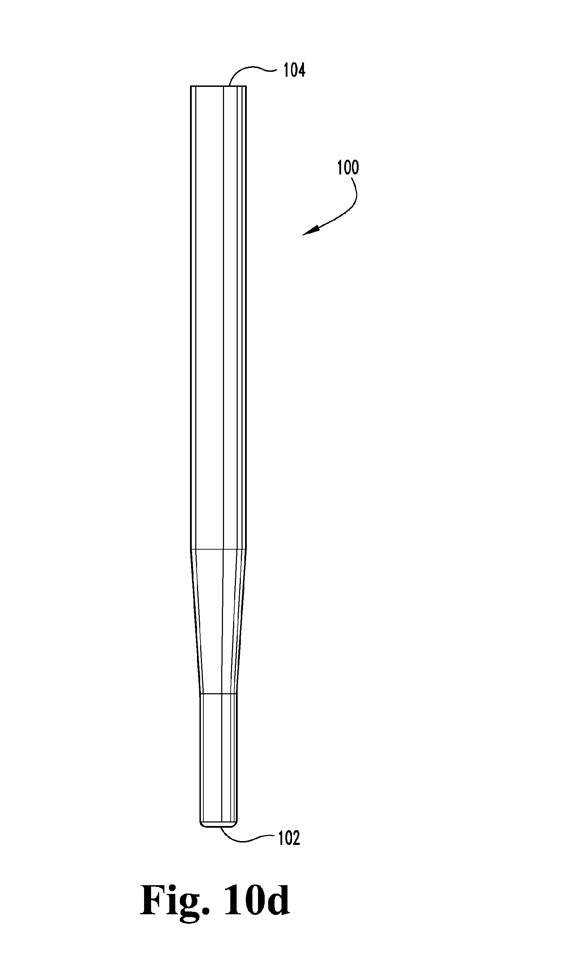

Referring to Figs. 10a-10d, a plurality of expansion tubes or members 100 are illustrated that are configured to laterally expand the blades 14 so that the incision 12 can be dilated to a predetermined size. Each expansion tube 100 includes a distal or insertion end 102 and a proximal end 104. Once the locking cap 24 is oriented in the unlocked state, the insertion end 102 of a respective one of the expansion tubes 100 is inserted through the opening 94 in the locking cap 24 and the hollow interior 92 of the main body 18 until an outside surface of the expansion tube 100 makes contact with the interiorly curved surfaces 96 of the blades 14. As illustrated in Figs. 10a-10d, the insertion ends 102 of the expansion tubes 100 have a smaller outside diameter than the proximal ends 104. As the outside diameter of the expansion tubes 100 increases the further the expansion tubes 100 are inserted or compressed down into the surgical dilator 10, the further out the blades 14 of the surgical dilator 10 laterally expand.

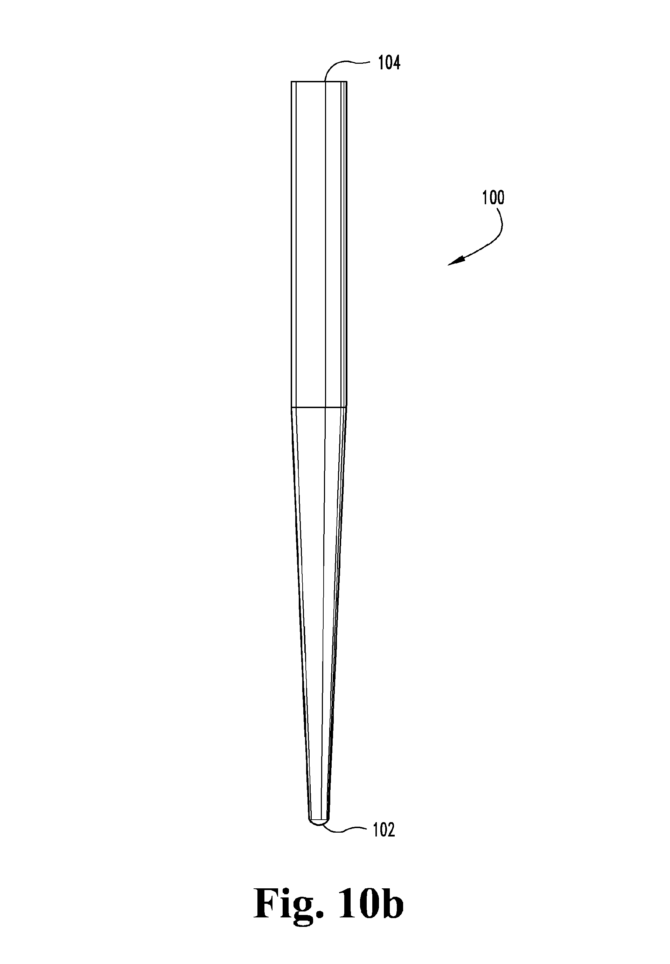

In the form illustrated in Fig. 10a, the expansion tube 10 is formed having a tapered stepped structure. The expansion tube 100 includes a plurality of steps 110 that increase in cross-sectional diameter as they progress from the insertion end 102 to the proximal end 104. As the steps 110 are inserted further into the surgical dilator 10, the steps 110 continuously make increasing contact with the interiorly curved surfaces 96 of the blades 14 thereby causing the blades 14 to laterally expand outwardly, which in turn causes the incision 12 to dilate further. In the forms illustrated in Figs. 10b-10d, the expansion tubes 100 are tapered outwardly as the body of the expansion tube 100 travels from the insertion end 102 to the proximal end 104. Again, the further the expansion tubes 10b-10d are inserted into the surgical dilator 10, the further the expansion tubes 100 cause the blades 14 to laterally expand. As one skilled in the art would recognize, the expansion tubes 100 illustrated in Figs. 10a-10d are for illustrative purposes only and various expansion tubes 100 having different predetermined expansion rates may be utilized herein.

As the expansion tubes 100 are inserted and make contact with the internally curved surfaces 96 of the blades 14, the blades 14 begin to expand laterally. As the blades 14 expand laterally, gaps 120 are formed between the respective blades 14 as illustrated in Fig. 2. However, because the expansion tubes 100 are positioned in contact with the interiorly curved surfaces 96 of the blades 14, the expansion tubes 100 prevent any tissue from creeping behind the blades 14. Any tissue that may attempt to position itself behind the blades 14 makes contact with the expansion tube 100, which prohibits the tissue from orienting itself behind the blades 14.

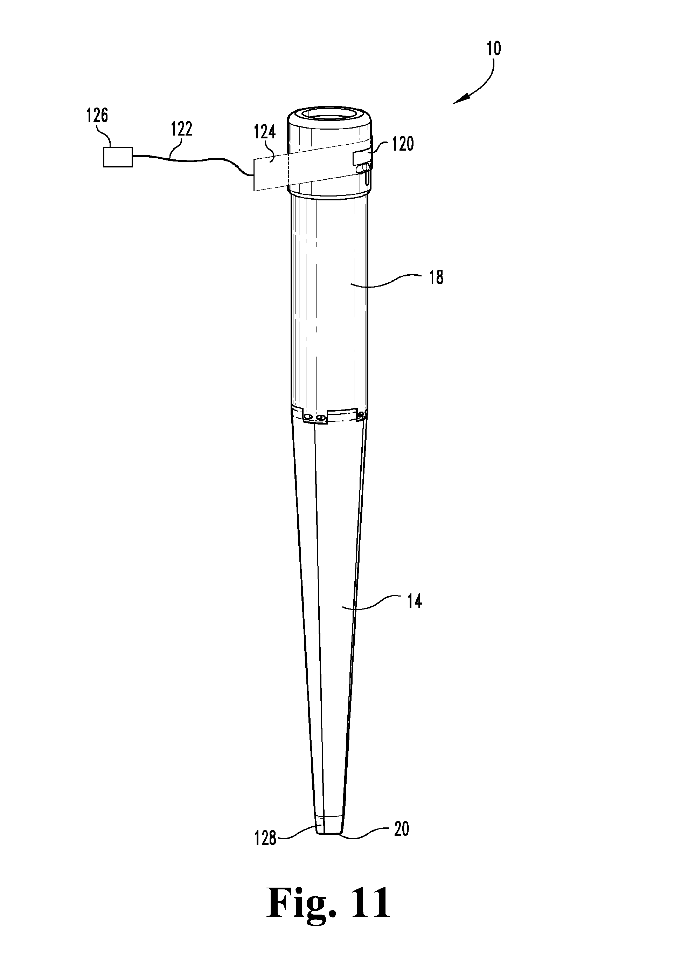

Referring to Fig. 11, in one representative form of the present invention the dilator 10 is provided with neuromonitoring capabilities. In this form, the dilator 10 can be substantially formed from a non-conductive material such as, for example, anodized aluminum. In this form, a portion of the locking cap 24 includes an area 120 that is conductive and exposed on an outer surface of the locking cap 24. The conductive area 120 permits the attachment of a stimulation signal transfer device 122 such as, for example, one or more wires to the locking cap 24. The stimulation signal transfer device 122 could be attached by way of a clip 124 or some other similar attachment device. The stimulation signal transfer device 122 is connected with an electric stimulation signal generator 126 that is operable to stimulate the dilator 10. The tip portion 20 of one or more of the blades 14 also includes a conductive area 128 exposed on an outer surface of the first dilation member 12. Neuromonitoring capability is achieved by stimulating the conductive area 128 of the tip 20 with electric signals to aid in detecting the proximity of the tip 20 to any neural structures. In other forms, the main body 18 could include the conductive area 120.

In one form, the surgical dilators disclosed herein are sized and configured to achieve a range of dilation from approximately 5.3mm to 21mm, but other ranges are envisioned. The dilation members and retaining pins disclosed herein can be manufactured from various materials such as aluminum, anodized aluminum, plastic, titanium, titanium alloys, steel, and so forth.

Although various embodiments have been described as having particular features and/or combinations of components, other embodiments are possible having a combination of any features and/or components from any of embodiments as discussed above. As used in this specification, the singular forms "a," "an" and "the" include plural referents unless the context clearly dictates otherwise. Thus, for example, the term "a member" is intended to mean a single member or a combination of members, "a material" is intended to mean one or more materials, or a combination thereof. Furthermore, the terms "proximal" and "distal" refer to the direction closer to and away from, respectively, an operator (e.g., surgeon, physician, nurse, technician, etc.) who would insert the medical implant and/or instruments into the patient. For example, the portion of a medical instrument first inserted inside the patient's body would be the distal portion, while the opposite portion of the medical device (e.g., the portion of the medical device closest to the operator) would be the proximal portion.

While the invention has been illustrated and described in detail in the drawings and foregoing description, the same is to be considered as illustrative and not restrictive in character, it being understood that all changes and modifications that come within the spirit of the invention are desired to be protected.

## Claims

1. A surgical dilator, comprising:
a main body having a distal end and a proximal end;
a locking cap connected with said proximal end of said main body operable to lock said surgical dilator in a closed state for insertion into an incision and unlock said surgical dilator to an open state after being inserted into said incision; and
a plurality of blades having a second proximal end pivotally connected with said distal end of said main body and extending downwardly toward a second distal end, wherein when said surgical dilator is in said closed state said blades taper downwardly and inwardly from said second proximal end toward said second distal end to form an insertion tip and when said surgical dilator is in said open state said blades are operable to laterally expand.

2. The surgical dilator of claim 1, further comprising an expansion tube sized to be inserted into an interior cavity defined by said main body and make contact with an interior surface of each said blade, wherein as said expansion tube is compressed into said interior cavity said expansion tube causes said blades to laterally expand and dilate said incision.

3. The surgical dilator of claim 1, further comprising a collar positioned between said proximal end of said main body and an end of said locking cap.

4. The surgical dilator of claim 3, further comprising a plurality of locking pins connected with a lower surface of said collar and extending downwardly through a plurality of channels formed in said main body.

5. The surgical dilator of claim 4, wherein in said open state a third distal end of each said locking pin is retracted away from said blades thereby allowing said blades to pivotally move in relation to said main body.

6. The surgical dilator of claim 3, further comprising
a plurality of locking pins connected with an end of said collar and positioned in a plurality of channels located in said main body, wherein a second distal end of said locking pins are operable to protrude outwardly from said channels on said distal end of said main body;
wherein said second distal ends of said locking pins are operable to engage an aperture in said blades to force said blades into a closed state for inserting said blades into an incision.

7. The surgical dilator of claim 6, wherein said second distal ends of said locking pins are operable to be released from said blades so that said blades are operable to laterally expand.

8. The surgical dilator of claim 6, further comprising a plurality of springs having a first portion positioned in a set of apertures located on said proximal end of said main body and a second portion in engagement with said end of said collar, wherein said springs are operable to force said locking cap upwardly thereby causing said distal ends of said locking pins to disengage said aperture in said blades thereby releasing said blades from said closed state.

9. The surgical dilator of claim 6, wherein said locking cap includes a conductive area and at least a lower portion of at least one of said blades include a second conductive area, wherein said second conductive area is configured to transmit an electric signal to provide said surgical dilator with neural monitoring capabilities.

10. The surgical dilator of claim 6, wherein said distal end of said main body includes a plurality of mounting arms and a third proximal end of each of said blades includes an arm, wherein said arms of said blades are oriented between said mounting arms of said main body and said arms are pivotally connected to said mounting arms with a plurality of pins.

11. The surgical dilator of claim 10, wherein said pins are inserted into said mounting arms and into said arms of said blades through a plurality of pin channels located on a side surface of said mounting arms.

12. The surgical dilator of claim 6, further comprising an expansion tube sized to be inserted into an interior cavity formed in said main body and into contact with an interior surface of said blades, wherein as said expansion tube is compressed downwardly said blades laterally expand when said blades are not in said closed state.

13. The surgical dilator of claim 1, further comprising:
means for orienting said blades in a locked state in which said blades combine to form a generally uniform blade structure having a generally cone-shaped configuration; and
means for orienting said blades in an unlocked state in which said blades can be laterally expanded.

14. The surgical dilator of claim 13, wherein said means for orienting said blades in said locked state comprises said locking cap connected with said proximal end of said main body, a collar positioned between said proximal end and said locking cap having a plurality of locking pins connected therewith that extend through a plurality of channels in said main body to engage said blades to orient said blades in said locked state.

15. The surgical dilator of claim 14, wherein said means for orienting said blades in said unlocked state comprises a control slot on a side surface of said locking cap and a pin extending through said control slot that is located on said main body, wherein when said pin is oriented in said control slot in a predetermined position said locking pins disengage said blades thereby allowing said blades to laterally expand.
